Europäisches Patentamt

European Patent Office

Office européen des brevets

(11) Publication number: **0 420 510 A2**

# EUROPEAN PATENT APPLICATION

(21) Application number: **90310328.1**

(22) Date of filing: **20.09.90**

(51) Int. Cl.5: **C07C 29/16, C07C 31/125**

(30) Priority: **28.09.89 GB 8921947**

(43) Date of publication of application:
**03.04.91 Bulletin 91/14**

(84) Designated Contracting States:
**BE DE ES FR GB IT NL**

(71) Applicant: **The British Petroleum Company p.l.c.**
**Britannic House Moor Lane**
**London EC2Y 9BU(GB)**

(72) Inventor: **Cole-Hamilton, David J.**
**University of St. Andrews**
**St. Andrews, Fife, KY16 9ST(GB)**
Inventor: **Macdougall, Joanna K.**
**University of St. Andrews**
**St. Andrews, Fife, KY16 9ST(GB)**
Inventor: **Green, Michael James**
**The British Petroleum Company p.l.c,**
**Chertsey Road**
**Sunbury-on-Thames, Middlesex TW16 7LN(GB)**

(74) Representative: **Barlow, Michael Thomas**
**BP INTERNATIONAL LIMITED Patents & Agreements Division Chertsey Road**
**Sunbury-on-Thames Middlesex TW16 7LN(GB)**

(54) **Process for the formation of alcohols.**

(57) A process for the preparation of alcohols comprises reacting one or more olefins with carbon monoxide and optionally hydrogen in the presence of a $C_1$ to $C_{20}$ aliphatic alcohol solvent and a catalyst comprising a phosphine component of formula $PR_3$ where R is independently $C_1$ to $C_{10}$ primary alkyl and a rhodium component. The solvent is preferably a primary aliphatic alcohol most preferably having from one to four carbon atoms.

## PROCESS FOR THE FORMATION OF ALCOHOLS

The present invention relates to a process for the formation of alcohols by the reaction of an olefin with carbon monoxide in the presence of a specific rhodium containing catalyst and an alcohol solvent.

Processes for the production of alcohols and aldehydes by reacting carbon monoxide with an olefin in the presence of a catalyst, are operated commercially. Systems based on cobalt catalysts eg $Co_2(CO)_8$ in the absence or presence of tertiary phosphines or rhodium catalysts eg [RhH(CO)-$(PPh_3)_3$] have been commercialised. It has been found however that in general cobalt catalysts, particularly in the presence of tertiary phosphines, give alcohol products while rhodium catalysts provide exclusively aldehyde products. Furthermore, reaction conditions used for rhodium are mild relative to the corresponding cobalt catalysed systems.

Rhodium catalysts have been used in processes for the provision of alcohol products although selectively has been poor and conditions extreme. For example US Patent 4438287 discloses a process for the production of alcohols using rhodium carbonyl complexes in the presence of amine promotors whilst the formation of alcohols using [eta $^5$-($C_5H_5$)Rh(CO)(PBu₃)] as a catalyst is discussed in Chem. Abst. No. 76,33787. The problem to be solved therefore is to produce alcohols from olefins and carbon monoxide using a rhodium catalyst under mild conditions.

We have now found that alcohols can be produced directly under milder conditions in such processes using a specific type of rhodium catalyst and a specific solvent.

Accordingly, the present invention comprises a process for the preparation of alcohols comprising reacting one or more olefins with carbon monoxide and optionally hydrogen in the presence of a $C_1$ to $C_{20}$ aliphatic alcohol solvent and a catalyst comprising a phosphine component of formula $PR_3$ where R is independently $C_1$ to $C_{10}$ primary alkyl and a rhodium component.

The present invention solves the problem defined above by providing a process which is carried out in the presence of a catalyst comprising a rhodium component and tris-alkyl phosphine ligands in the presence of a lower aliphatic alcohol solvent. The process of the present invention can be carried out under mild conditions and selectivity to give the alcohol products in high yield.

The olefin may suitably be any olefin having 2 to 30 carbon atoms per molecule. The olefin may have one or more carbon-carbon double bonds, and the carbon-carbon double bond(s) may be internal or terminal. Preferred olefins are those represented by the general formula $R^2CH = CHR^1$ where both $R^2$ and $R^1$ are independently selected from H, $C_1$ to $C_{10}$ alkyl, $C_1$ to $C_{10}$ alkenyl, or $C_6$ to $C_{12}$ aryl or alkyl substituted derivatives. Examples of such compounds are but-1-ene, but-2-ene, pent-1-ene, hex-1-ene, hex-2-ene, hex-3-ene, oct-1-ene and dec-1-ene. Most preferred olefins are terminal olefins ie those where $R^1$ is H.

The process of the present invention proceeds so that the alcohol produced has one carbon atom more than the olefin used to produce it.

The carbon monoxide and hydrogen may be derived from any suitable source. An elevated pressure, suitably in the range 0.5 -15 MPa, preferably 1-8 MPa, at the reaction temperature is employed. The reaction temperature is suitably 50-200°C, preferably 70-160°C. If hydrogen is used a suitable partial pressure is one in the range 0-15 MPa.

The reaction is carried out in the presence of a $C_1$ to $C_{20}$ aliphatic alcohol solvent, preferably $C_1$ to $C_{10}$, most preferably $C_1$ to $C_4$. The aliphatic alcohols used are preferably primary aliphatic alcohols eg methanol, ethanol n-propanol and n-butanol.

The phosphine component of the catalyst is one having the general formula $PR_3$ where the R groups are independently selected from $C_1$ to $C_{10}$ primary alkyl, preferably $C_1$ to $C_4$ primary alkyl. Most preferred are those where all three R groups are the same. An example of a preferred phosphine is triethyl phosphine.

Considering the rhodium component, this can be derived from any rhodium source eg $Rh_2OAc_4$ or simple inorganic rhodium salts. Alternatively a rhodium/phosphine complex can be used. This latter approach of course means that some or all of the phosphine component will be supplied with the rhodium component. In this respect, suitable materials are the complexes having the formula [HRh-($PR_3$)₃] where the R groups are as defined above.

It is preferred to employ amounts of each component such that the mole ratio of rhodium to phosphine component is at least 1:1 preferably between 1:1 and 1:100.

The process can be carried out either batchwise or continously.

The process of the present invention is now described with reference to the following examples.

Example 1

A glass lined autoclave was charged with 1.2cm³ hexene, 4.8cm³ ethanol and 0.02g of

[HRhP(Et$_3$)$_3$] under a nitrogen atmosphere. The autoclave was sealed and then purged with carbon monoxide. The vessel was pressured, using a 1:1 mixture of carbon monoxide and hydrogen to 8 MPa and thereafter heated with stirring to 120°C. The reaction temperature was maintained at 120°C for four hours. The autoclave was then cooled, opened and the product analysed using gas liquid chromatography. The yield of 75.4% C$_7$ alcohols was obtained with a ratio of normal to iso of 2.02:1 and a yield of 1% aldehyde.

## Example 2

The procedure for Example 1 was repeated except 0.022g of rhodium component was used. The vessel was pressurised to 6.5 MPa and the reaction carried out over 16 hours. A yield of 100% C$_7$ alcohols was obtained with a ratio of normal to iso of 2.07:1.

## Example 3

The procedure of Example 2 was repeated except that the vessel was pressurised to 3 MPa. A yield of 97% C$_7$ alcohol vas obtained with a ratio of normal to iso alcohols of 1.93:1.

## Example 4

The procedure of Example 1 was repeated except that 60mg of PEt$_3$ was also added to the autoclave prior to sealing. The vessel was then pressurised to 3 MPa and reaction conditions maintained for 16 hours. A yield of 93.9% of C$_7$ alcohols was obtained, having a ratio of normal to iso of 2.20:1.

## Example 5

The procedure of Example 4 was repeated except that the reaction was carried out over five hours. A yield of 63.3% C$_7$ alcohols was obtained. The ratio of normal to iso alcohol was 2.6:1.

## Example 6

A glass lined autoclave was charged with 1.2cm$^3$ hexene, 4.8cm$^3$ ethanol, 0.018g of [Rh$_2$OAc$_4$] and 20mg of PEt$_3$ under a nitrogen atmosphere. The vessel was sealed and purged with carbon monoxide. The pressure was set to 5.9 MPa using a 1:1 mixture of carbon monoxide and hydrogen. The vessel was heated to 120°C and reaction conditions maintained for 16.5 hours. The vessel was then cooled, opened and the contents analysed by gas liquid chromatography. A yield of 100 C$_7$ alcohols was obtained. The ratio of normal to iso alcohol was 2.23:1.

## Example 7

The procedure of Example 6 was repeated except that PMe$_3$(10mg) was used instead of PEt$_3$. The vessel was pressurised to 4.8 MPa and the reaction conditions maintained for 16 hours. A yield of 99.1% C$_7$ alcohols was obtained. The ratio of normal to iso alcohol was 2.54:1.

## Example 8

The procedure of Example 6 was repeated except that PBu$_3$( 30mg) was used instead of PEt$_3$. The vessel was pressurised to 4.4 MPa and reaction conditions maintained for 16 hours. A yield of 99.4% C$_7$ alcohols was obtained. The ratio of normal to iso alcohol was 2.45:1.

## Example 9

A glass lined autoclave was charged with 1cm$^3$ of hexene, 4cm$^3$ of methanol, 60mg of PEt$_3$ and 0.02g of [HRh(PEt$_3$)$_3$] under a nitrogen atmosphere. The autoclave was closed, pressurised to 3MPa with carbon monoxide and the contents thereafter heated with stirring to 144°C. The reaction conditions were maintained for 16 hours. The vessel was then cooled, opened and the products analysed by gas liquid chromatography. A yield of 73.5% C$_7$ alcohol was obtained with normal to iso ratio of 1.4:1.

## Example 10

The procedure of Example 9 was repeated except that the alcohol solvent was ethanol. A yield of 66.3% C$_7$ alcohol was obtained with normal to iso ratio of 1.7:1.

## Example 11

The procedure of Example 9 was repeated except the alcohol solvent was n-butanol. A yield of 49.5% C$_7$ alcohol was obtained with normal to iso ratio of 2.9:1.

Comparative Example 1

A glass lined autoclave was charged with $1.2cm^3$ hexene, $4.8cm^3$ toluene and 0.02g of $[HRhP(Et_3)_3]$ under a nitrogen atmosphere. The autoclave was sealed and purged with carbon monoxide. The vessel was pressurised using a 1:1 mixture of carbon monoxide and hydrogen to 8 MPa and the contents thereafter heated with stirring to 120°C. The reaction conditions were maintained for 16 hours. The vessel was then cooled, opened and the products analysed by gas liquid chromatography. A yield of 13.4% $C_7$ alcohols and 74.7% $C_7$ aldehydes was obtained. The ratio of normal to iso alcohol was 3.32:1.

**Claims**

1. A process for the preparation of alcohols comprising reacting one or more olefins with carbon monoxide and optionally hydrogen in the presence of a $C_1$ to $C_{20}$ aliphatic alcohol solvent and a catalyst comprising a phosphine component of formula $PR_3$ where R is independently $C_1$ to $C_{10}$ primary alkyl and a rhodium component.

2. A process as claimed in claim 1 where the solvent is a $C_1$ to $C_4$ primary aliphatic alcohol.

3. A process as claimed in claim 1 wherein the phosphine component has a general formula $PR_3$ where R is independently $C_1$ to $C_4$ primary alkyl.

4. A process as claimed in claim 3 wherein the phosphine component is $PEt_3$.

5. A process as claimed in claim 1 wherein the rhodium component is, or is derived from $RhH(PEt_3)_3$.

6. A process as claimed in claim 1 wherein the molar ratio of phosphine component to rhodium component is 1:1 to 1:100.

7. A process as claimed in claim 1 wherein the olefin is represented by the general formula $R^2CH = CHR^1$ where both $R^2$ and $R^1$ are independently selected from H, $C_1$ to $C_{10}$ alkyl, $C_1$ to $C_{10}$ alkenyl and $C_6$ to $C_{12}$ aryl or alkyl substituted derivatives thereof.